# EUROPEAN PATENT APPLICATION

(11) **EP 4 238 995 A1**
(43) Date of publication of application: **06.09.2023**
(21) Application number: 21886638.2
(22) Date of filing: 15.10.2021
(51) Int. Cl.: C07K 16/40, A61P 31/00, G01N 33/569, A61K 39/00

(54) **WARS-NEUTRALIZING ANTIBODY AND USE THEREOF**

(30) Priority: 27.10.2020 KR 20200140371; 14.10.2021 KR 20210136547
(71) Applicant: MirimGENE Co., Ltd., 21999 Incheon (KR)
(72) Inventor: JIN, Mirim, Seoul 05649 (KR); CHOI, Yun Hui, Inceon 21910 (KR)
(74) Representative: V.O.
(86) International application number: PCT/KR2021/014301
(87) International publication number: WO 2022/092644

(57) **Abstract**

The present disclosure relates to an antibody binding specifically to tryptophanyl-tRNA synthetase (WARS) or a fragment thereof. The antibody or the fragment thereof of the present disclosure has an excellent ability to bind specifically to WARS with no cross-reactivity with other ARSs and has an excellent ability to detect WARS and inhibit the activity thereof. Therefore, it can be effectively used for diagnosing and/or treating an infectious disease.

## Description

### [Technical Field]

The present disclosure relates to an antibody that binds specifically to the WARS protein and a use thereof.

### [Background Art]

Aminoacyl-tRNA synthetase (ARS) is an enzyme that attaches an amino acid specifically onto a tRNA and plays a critical role in the production of proteins. Recently, it has been known that ARS is involved in various biological phenomena such as apoptosis, angiogenesis, inflammatory responses, etc. in addition to its essential functions. Especially, human tryptophanyl-tRNA synthetase 1 (WARS1), which is an aminoacyl-tRNA synthetase that recognizes tryptophan and attaches it to a tRNA for protein synthesis, has been recently known to play a role in the host defense mechanism against infection. The inventors of the present disclosure have previously reported that WARS1 is promptly secreted by monocytes into the extracellular space within several minutes following infection without *de novo* synthesis (Young Ha Ahn. et al. Secreted tryptophanyl-tRNA synthetase as a primary defense system against infection. Nature Microbiology 2: 16191 (2016)).

Functioning as an endogenous ligand of TLR4 and TLR2, the secreted WARS1 induces activation of innate immunity by activating macrophages. In addition, the WARS1 initiates the production of proinflammatory cytokines and chemokines such as TNF-α, IL-6, MIP-1α, IL-8 and IFN-γ and induces the infiltration of neutrophils. Subsequently, the WARS1 is secreted continuously as the expression of the WARS1 gene is activated by IFN-γ. Congruously with these findings, the WARS1 is highly enriched in the blood of septic patients but not in aseptic chronic inflammatory disorders. It is secreted regardless of pathogen types such as gram-positive and negative bacteria, viruses, fungi, etc.

For treatment of infectious inflammatory diseases caused by bacteria, virus and/or fungi, a strategy using antibodies may be considered. However, since ARSs including WARS1 are similar in protein structures, the antibodies tend to show low sensitivity because of cross-reactivity with other ARSs.

Accordingly, it is necessary to develop an antibody that can effectively neutralizing WARS1 by binding specifically only thereto without cross-reaction with other ARSs.

The description given in the Background Art section is provided only to enhance the understanding of the background of the present disclosure and should not be construed as recognizing that it is well known to those having ordinary knowledge in the art.

### [Disclosure]

### [Technical Problem]

The inventors of the present disclosure have made consistent efforts to find out an antibody binding specifically to WARS, the expression level is increased remarkably in a patient with an infectious disease such as sepsis, or a fragment thereof. As a result, they have found an antibody having an excellent ability to bind specifically to WARS with no cross-reactivity with other ARSs and having an excellent ability to detect WARS and inhibit the activity thereof, and have completed the present disclosure.

The present disclosure is directed to providing an antibody binding specifically to tryptophanyl-tRNA synthetase (WARS) or a fragment thereof.

The present disclosure is also directed to providing a polynucleotide encoding the antibody or a fragment thereof.

The present disclosure is also directed to providing a vector including the polynucleotide.

The present disclosure is also directed to providing a cell transformed with the vector.

The present disclosure is also directed to providing an antibody binding specifically to WARS or a fragment thereof, which includes a step of culturing the cell under a condition where the polynucleotide is expressed and recovering the polypeptide.

The present disclosure is also directed to providing a method for detecting WARS specifically, which includes a step of contacting the antibody or a fragment thereof, the polynucleotide, the vector or the cell with a sample.

The present disclosure is also directed to providing a composition for detecting WARS specifically, which contains the antibody or a fragment thereof, the polynucleotide, the vector or the cell.

The present disclosure is also directed to providing a composition for diagnosing an infectious disease, which contains the antibody or a fragment thereof, the polynucleotide, the vector or the cell.

The present disclosure is also directed to providing a pharmaceutical composition for preventing or treating an infectious disease, which contains the antibody or a fragment thereof, the polynucleotide, the vector or the cell.

The present disclosure is also directed to providing a pharmaceutical composition for preventing or treating an infectious disease, which contains the antibody or a fragment thereof, the polynucleotide, the vector or the cell and additionally an antibiotic.

Other purposes and advantages of the present disclosure will become more apparent by the following detailed description, claims and drawings.

### [Technical Solution]

In an aspect, the present disclosure provides an antibody binding specifically to tryptophanyl-tRNA synthetase (WARS) or a fragment thereof.

The inventors of the present disclosure have made efforts to find out an antibody binding specifically to WARS, the expression level of which is increased remarkably in a patient with an infectious disease such as sepsis, or a fragment thereof. As a result, they have found an antibody having an excellent ability to bind specifically to WARS with no cross-reactivity with other ARSs and having an excellent ability to detect WARS and inhibit the activity thereof.

In the present specification, the term "WARS" refers to tryptophanyl-tRNA synthetase. It is also known as tryptophan-tRNA ligase, TrpRS, WRS, etc. WARS is an enzyme which mediates the aminoacylation reaction between the amino acid tryptophan and a tRNA. In human, WARS is encoded by the WARS gene. The protein's amino acid sequence and mRNA base sequence are known as Genbank accession number NP_004175.2 (protein) and Genbank accession number NM_004184.3 (mRNA base sequence). WARS has two isoforms: cytoplasmic form (WARS1 or tryptophanyl-tRNA synthetase 1, cytoplasmic) and mitochondrial form (WARS2 or tryptophanyl-tRNA synthetase 2, mitochondrial).

According to a specific exemplary embodiment of the present disclosure, the WARS of the present disclosure is WARS1.

In the present specification, the term "antibody" refers to a molecule having an antigen-binding site for specific binding to an antigen. As used in the present specification, the term includes a full-length antibody, any fragment thereof (i.e., an "antigen-binding moiety") or a single chain thereof. According to an exemplary embodiment of the present disclosure, the "antibody" refers to a glycoprotein including at least two heavy chains (H) and two light chains (L) connected by disulfide bonds or an antigen-binding moiety thereof. According to another exemplary embodiment of the present disclosure, the "antibody" refers to a single-chain antibody including a single variable domain, e.g., a V_{H} domain. Each heavy chain consists of a heavy chain variable domain (V_{H}) and a heavy chain constant domain. In general, the heavy chain constant domain includes three domains, C_{H}1, C_{H}2 and C_{H}3, and each light chain includes a light chain variable domain (abbreviated as V_{L}) and a light chain constant domain. The light chain constant domain includes one domain, i.e., C_{L}.

The V_{H} and V_{L} domains can be further subdivided into hypervariable regions, referred to as complementarity-determining regions (CDRs), which are interposed between more conserved regions called framework regions (FRs). Each V_{H} and V_{L} is composed of three CDRs and four FRs, which are arranged from the amino-terminus to the carboxyl-terminus in the order of FR1, CDR1, FR2, CDR2, FR3, CDR3 and FR4. The variable domain of the heavy chain and the light chain includes a binding domain that interacts with an antigen. The constant domain of the antibody may mediate the binding of the immunoglobulin to host tissues or factors, including various cells of the immune system (e.g., effector cells) and the first component of the classical complement system (C1q).

In the present specification, the term "Fc" refers to the C-terminal region of the antibody heavy chain which mediates the binding of the immunoglobulin to host tissues or factors, including the FC receptors on various cells of the immune system (e.g., effector cells) and the first component of the classical complement system (C1q). Accordingly, the Fc includes the constant region of the antibody excluding the first constant region immunoglobulin domain (e.g., C_{H}1 or C_{L}). In IgG, IgA and IgD antibody isotypes, the Fc region includes two identical protein fragments derived from the second (C_{H}2) and third (C_{H}3) constant domains of the antibody's two heavy chains. In IgM and IgE, the Fc region includes three heavy chain constant domains (C_{H} domain 2-4) in each polypeptide chain. In the present specification, the Fc may be a native sequence including any allotypic variant or an Fc variant (e.g., non-naturally occurring Fc). In addition, the Fc may be an Fc-containing protein polypeptide such as "Fc region-region containing fusion protein", also called an "Fc fusion protein" (e.g., an antibody or immunoadhesin).

The antibody can be of any type (e.g., IgG, IgE, IgM, IgD, IgA or IgY) or any subclass (e.g., IgG1, IgG2, IgG3 or IgG4 in human, and IgG1, IgG2a, IgG2b or IgG3 in mouse) of immunoglobulin molecules. The immunoglobulin, e.g., IgG1, exists as several allotypes which differ from each other in a few amino acids. The antibody disclosed in the present specification may be derived from any of the commonly known isotypes, classes, subclasses or allotypes. In a specific exemplary embodiment, the antibody presented in the present specification belongs to the IgG1, IgG2, IgG3 or IgG4 subclass or any hybrid thereof. In a specific exemplary embodiment, the antibody belongs to the IgG2, IgG4 or IgG2/IgG4 subclass.

The antibody includes, by way of examples, naturally occurring or non-naturally occurring antibodies; monoclonal or polyclonal antibody; chimeric or humanized antibodies; human or non-human antibodies; wholly synthetic antibodies; single-chain antibody; monospecific antibodies; and multispecific antibodies (including bispecific or trispecific antibodies).

In the present specification, the term "neutralizing antibody" refers to an antibody that defends a cell from a pathogen or infectious particle by neutralizing any effect it has biologically. The neutralizing antibody is a part of the adaptive immune response of against viruses, bacteria, fungi and microbial toxins. The neutralizing antibody achieves immunity by binding to the surface structure of the infectious particle and thereby preventing the infectious antigen from interacting with the host cell.

In the present specification, the term "fragment" is understood to include all forms such as one or more fragment of an antibody having the ability of binding specifically to an antigen or the "one or more fragment of an antibody having the ability of binding specifically to an antigen" bound to another molecule (including a part (e.g., Fc) of the same or different antibody). Examples of the fragment include a monovalent fragment (Fab fragment) consisting of V_{L}, V_{H}, C_{L} and C_{H}1 domains; a bivalent fragment (F(ab')₂ fragment) including two Fab fragments linked by a disulfide bridge at the hinge region; an Fd fragment consisting of V_{H} and C_{H}1 domains; an Fv fragment consisting of the V_{L} and V_{H} domains of a single arm of an antibody and disulfide-linked Fv (sdFv); a dAb fragment consisting of a V_{H} domain; and an isolated complementarity-determining region (CDR) or a combination of two or more isolated CDRs which can be optionally be joined by a linker. Furthermore, V_{L} and V_{H} regions may be joined by a linker so that they are paired as a single protein chain to form a monovalent molecule (single-chain Fv (scFv)). These single-chain antibodies are also included in the antibody fragments. In addition, the antibody or antibody fragment includes tetrameric antibodies including two heavy chain molecules and two light chain molecules; antibody light chain monomers; antibody heavy chain monomers; antibody light chain dimers, antibody heavy chain dimers; intrabodies; monovalent antibodies; camelized antibodies; and single-domain antibodies (sdAbs).

According to a specific exemplary embodiment of the present disclosure, the antibody fragment of the present disclosure is a fragment selected from a group consisting of Fab, F(ab')₂, Fd, sdFv, Fv, dAb, scFv, sdAb and a tetramer or the fragment bound to Fc.

According to a specific exemplary embodiment of the present disclosure, the antibody or the fragment thereof of the present disclosure includes a heavy chain variable domain (V_{H}) including a heavy chain CDR1 of SEQ ID NO 3, a heavy chain CDR2 of SEQ ID NO 4 and a heavy chain CDR3 of SEQ ID NO 5.

According to a specific exemplary embodiment of the present disclosure, the antibody or the fragment thereof of the present disclosure includes a light chain variable domain (V_{L}) including a light chain CDR1 of SEQ ID NO 6, a light chain CDR2 of SEQ ID NO 7 and a light chain CDR3 of SEQ ID NO 8.

According to a specific exemplary embodiment of the present disclosure, the antibodies (including the fragments thereof) of the present disclosure having the CDR sequences described above bind specifically to WARS with no cross-reactivity with other proteins including the ARS family.

According to a specific exemplary embodiment of the present disclosure, the antibody or the fragment thereof of the present disclosure includes a heavy chain variable domain (V_{H}) of SEQ ID NO 1 and a light chain variable domain (V_{L}) of SEQ ID NO 2.

According to a specific exemplary embodiment of the present disclosure, the antibody or the fragment thereof of the present disclosure includes one or more sequence selected from a group consisting of a C_{L} sequence of SEQ ID NO 21, a C_{H}1 sequence of SEQ ID NO 22, a hinge sequence of SEQ ID NO 23, a C_{H}2 sequence of SEQ ID NO 24 and a C_{H}3 sequence of SEQ ID NO 25.

According to a specific exemplary embodiment of the present disclosure, the antibody or the fragment thereof of the present disclosure includes all of the C_{L} sequence of SEQ ID NO 21, the C_{H}1 sequence of SEQ ID NO 22, the hinge sequence of SEQ ID NO 23, the C_{H}2 sequence of SEQ ID NO 24 and the C_{H}3 sequence of SEQ ID NO 25.

In another aspect, the present disclosure provides a polynucleotide encoding the antibody or the fragment thereof, a vector including the same polynucleotide or a cell transformed with the vector.

In the present specification, the term "polynucleotide" or "nucleic acid molecule" includes a DNA molecule and an RNA molecule. The polynucleotide or nucleic acid molecule of the present disclosure may be isolated or recombinant and includes not only the DNA and RNA in the form of a single chain or a double chain but also a sequence complementary thereto. When the "isolated nucleic acid" is a nucleic acid isolated from a naturally occurring source, it is a genomic nucleic acid isolated from an individual. For a nucleic acid synthesized enzymatically or chemically from a template, such as a PCR product, a cDNA molecule or an oligonucleotide, a nucleic acid produced therefrom may be understood as an isolated nucleic acid molecule. The isolated nucleic acid molecule refers to a nucleic acid molecule in the form of a separate fragment or as a component of a larger nucleic acid construct. The nucleic acid is "operably linked" when it is placed into a functional relationship with another nucleic acid sequence. For example, a DNA of a presequence or a secretory leader is operably linked to a DNA of a polypeptide if it is expressed as a preprotein before secretion of the polypeptide; a promoter or an enhancer is operably linked to a coding sequence if it affects the transcription of the polypeptide sequence; or a ribosomal binding site is operably linked to a coding sequence so as to facilitate translation. Generally, "operably linked" means that the DNA sequences being linked are contiguous and, in the case of a secretory leader, are contiguous and in the same reading frame. However, enhancers do not have to be contiguous. The linking is accomplished by ligation at convenient restriction enzyme sites. If such sites do not exist, a synthetic oligonucleotide adapter or linker is used in accordance with conventional practice.

In the present specification, the term "vector" refers to a carrier capable of inserting a polynucleotide sequence for introduction into a cell that can replicate the polynucleotide (nucleic acid) sequence. The polynucleotide sequence may be exogenous or heterologous. The vector may be a plasmid, a cosmid vector or a viral vector (e.g., a retroviral, adenoviral or adeno-associated viral vector), although not being limited thereto. Those skilled in the art can establish the vector according to a standard recombinant technique (Maniatis, et al., Molecular Cloning, A Laboratory Manual, Cold Spring Harbor Press, Cold Spring Harbor, N.Y., 1988; Ausubel et al., In: Current Protocols in Molecular Biology, John, Wiley & Sons, Inc, NY, 1994).

In the present specification, the term "expression vector" refers to a vector including a nucleotide sequence encoding at least a part of a gene product being transcribed. In some cases, an RNA molecule is then translated into a protein, a polypeptide or a peptide. The expression vector may include various regulatory sequences. In addition to the regulatory sequence that regulates transcription and translation, the expression vector may also include nucleic acid sequences that provide different functions.

In the present specification, the term "cell" includes a eukaryotic cell and a prokaryotic cell and refers to any transformable cell capable of replicating the vector or expressing a gene encoded by the vector. The cell may be transfected, transduced or transformed by the vector, which refers to a process whereby an exogenous polynucleotide (nucleic acid molecule) is delivered or introduced into a host cell. In the present specification, the term "transformation" is used to encompass the meaning of transfection and transduction.

The (host) cell of the present disclosure may be specifically a bacterial cell, an insect cell or a mammalian cell, more specifically an insect cell such as Sf9 cells, an mammalian cell such as HEK293 cells, HeLa cells, ARPE-19 cells, RPE-1 cells, HepG2 cells, Hep3B cells, Huh-7 cells, C8D1a cells, Neuro2A cells, CHO cells, MES13 cells, BHK-21 cells, COS7 cells, COP5 cells, A549 cells, MCF-7 cells, HC70 cells, HCC1428 cells, BT-549 cells,PC3 cells, LNCaP cells, Capan-1 cells, Panc-1 cells, MIA PaCa-2 cells, SW480 cells, HCT166 cells, LoVo cells, A172 cells, MKN-45 cells, MKN-74 cells, Kato-III cells, NCI-N87 cells, HT-144 cells, SK-MEL-2 cells, SH-SY5Y cells, C6 cells, HT-22 cells, PC-12 cells, NIH3T3 cells, etc., although not being limited thereto.

The (host) cell of the present disclosure may be specifically a cell which is not an embryonic stem cell.

According to a specific exemplary embodiment of the present disclosure, the host cell of the present disclosure is an isolated or separated host cell.

In another aspect, the present disclosure provides a method for preparing an antibody binding specifically to WARS or a fragment thereof, which includes a step of culturing the cell under a condition where the polynucleotide is expressed and recovering the polypeptide.

The antibody or a fragment thereof, the nucleotide, the vector, the cell, etc. are the same as described above. The polypeptide may be or include the antibody or the fragment thereof (antigen-binding fragment) of the present disclosure.

According to a specific exemplary embodiment of the present disclosure, the method of the present disclosure includes: (a) a step of transforming a host cell with an expression vector (recombinant expression vector) including the polynucleotide; (b) a step of producing an antibody or a fragment thereof by culturing the transformed host cell; and (c) a step of host cell obtaining the produced antibody or the fragment thereof.

In another aspect, the present disclosure provides a method for detecting WARS specifically, which includes a step of contacting the antibody or a fragment thereof, the polynucleotide, the vector or the cell with a sample.

In another aspect, the present disclosure provides a composition for detecting WARS specifically, which contains the antibody or a fragment thereof, the polynucleotide, the vector or the cell.

The antibody or the fragment thereof of the present disclosure is useful for diagnostic assay for detecting and quantifying the expression of the WARS protein in, for example, a specific cell, tissue or serum, because it binds specifically to WARS.

In the present specification, the term "sample" may be a cell, tissue, blood, whole blood, serum, plasma, cerebrospinal fluid, etc. taken from a subject (e.g., a subject to be diagnosed for an infectious disease or an infectious complication). The detection of the protein using the antibody or a fragment thereof, the polynucleotide, the vector or the cell may be conducted by western blot, immunoblot, dot blot, immunohistochemistry, enzyme-linked immunosorbent assay (ELISA), radioimmunoassay, competitive binding assay, immunoprecipitation, etc., although not being limited thereto.

In another aspect, the present disclosure provides a composition for diagnosing an infectious disease, which contains the antibody or a fragment thereof, the polynucleotide, the vector or the cell.

In another aspect, the present disclosure provides a kit for diagnosing an infectious disease, which includes the antibody or a fragment thereof, the polynucleotide, the vector or the cell.

In another aspect, the present disclosure provides a pharmaceutical composition for preventing or treating an infectious disease, which contains the antibody or a fragment thereof, the polynucleotide, the vector or the cell.

In another aspect, the present disclosure provides a pharmaceutical composition for treating a disease caused by overexpression of WARS, which contains the antibody or a fragment thereof, the polynucleotide, the vector or the cell.

The antibody, the fragment thereof, the nucleotide, the vector, the cell, etc. are the same as described above.

The disease to be prevented, alleviated or treated by the present disclosure includes but is not limited to any disease associated with the expression or activation of WARS.

Specifically, the disease associated with the expression or activation of WARS includes an infectious disease, although not being limited thereto.

The expression level of WARS is increased rapidly from the early stage of infection by bacteria, virus, fungi, etc. It has been reported that the expression is increased significantly as compared to a normal control group when the symptoms of pneumonia or sepsis occur due to infectious complication. Furthermore, the expression level WARS is highly correlated with the severity and prognosis of sepsis in septic patients and WARS is increased only in infectious inflammations. Therefore, WARS is very valuable as a diagnostic marker for new infectious diseases and infectious complications because infectious inflammatory diseases and noninfectious inflammatory diseases can be distinguished quickly and clearly. In particular, the level of WARS in serum is increased remarkably in the patients with sepsis or septic shock caused by infection by bacteria or fungi as compared to a healthy normal control group, whereas the increase of WARS is not statistically significant in septic patients infected by gram-negative bacteria, gram-positive bacteria or fungi. Therefore, WARS can be usefully used for the diagnosis of sepsis caused by infection by gram-negative bacteria, gram-positive bacteria or fungi. In addition, the level of WARS is increased more in patients with septic shock than in patients with sepsis and the expression level of WARS is associated with the severity of sepsis. That is to say, it can be said that the symptoms of sepsis are severe as the expression level of WARS is higher (Korean Patent Publication No. 10-2017-0027313). That is to say, when the WARS-specific antibody of the present disclosure is used, the expression level of WARS in a biological sample can be detected with high accuracy and, therefore, information useful for diagnosing infectious diseases and infectious complications and predicting the prognosis thereof can be provided.

The antibody or the fragment thereof according to the present disclosure can be provided as an assay kit or a diagnostic kit. The kit is not specially limited as long as it is an assay kit providing an antibody or a peptide having a specific binding domain. For example, it may be a kit for western blot, ELISA, radioimmunoassay, radial immunodiffusion, Ouchterlony immunodiffusion, rocket immunoelectrophoresis, immunohistochemistry, immunoprecipitation assay, complement fixation assay, FACS or protein chip assay.

The infectious disease of the present disclosure may be pneumonia, phthisis, tuberculosis, sepsis, septic shock, salmonellosis, food poisoning, typhoid fever, urinary tract infection, cystitis, pyelonephritis, urethritis, prostatitis, upper respiratory infection, otitis media and infection by acquired immunodeficiency syndrome (AIDS) virus, coronavirus, etc., although not being limited thereto.

The coronavirus includes sever acute respiratory syndrome-related coronavirus (SARS-CoV), Middle East syndrome-related coronavirus (MERS-CoV), SARS-related coronavirus 2 (SARS-CoV-2) which is the cause of COVID-19 (coronavirus disease 2019), infectious bronchitis virus (IBV), transmissible gastroenteris virus (TGEV), porcine epidemic diarrhea virus (PEDV), bovine coronavirus (BCV), feline/canine coronavirus (FCoV/CCoV), mouse hepatitis virus (MHV), etc., although not being limited thereto.

According to a specific exemplary embodiment of the present disclosure, the infectious disease is an infectious inflammatory disease.

The infectious inflammatory disease is an infectious disease accompanied by inflammation and an anticipated therapeutic effect may be achieved by inhibiting the proliferation of pathogens (virus, bacteria, fungi, etc.) which cause the inflammation or regulating the inflammation.

According to a specific exemplary embodiment of the present disclosure, the infectious inflammatory disease is sepsis or septic shock.

The sepsis includes early-onset sepsis, severe sepsis, septic shock and multiple organ dysfunction syndrome (MODS), disseminated intravascular coagulation (DIC), acute respiratory distress syndrome (ARDS) or acute kidney injury (AKI) accompanied by sepsis, although not being limited thereto.

The composition of the present disclosure is advantageous in that it can not only detect WARS with high sensitivity but also significantly reduce the amount of the WARS increased due to infection and can achieve diagnosis and treatment at the same time by lowering the expression level of inflammatory cytokines (e.g., IL-8, IL-6, MIP-1α, TNF-α, etc.).

In the present specification, the term "cytokine" refers to a protein immunomodulator secreted from immune cells, and includes a chemokine.

The cytokine includes IL-8, IL-6, MIP-1α, INF-γ, TNF-α, IL-1β, IL-10, etc.

According to a specific exemplary embodiment of the present disclosure, the pharmaceutical composition contains a pharmaceutically acceptable carrier.

According to a specific exemplary embodiment of the present disclosure, the pharmaceutical composition of the present disclosure further contains an antibiotic in addition to the antibody or a fragment thereof, the polynucleotide, the vector or the cell.

Specifically, the antibiotic includes gentamycin, ampicillin, kanamycin, chloramphenicol, streptomycin, tetracycline, erythromycin, vancomycin, penicillin, spectinomycin, sulfadiazine and trimethoprim, although not being limited thereto.

According to an exemplary embodiment of the present disclosure, the survival rate of a subject having an infectious disease may be increased by administering the WARS-specific antibody of the present disclosure in combination with an antibiotic or before or after administering an antibiotic.

The pharmaceutically acceptable carrier contained in the pharmaceutical composition of the present disclosure includes, as ingredients commonly used for formulation, lactose, dextrose, sucrose, sorbitol, mannitol, starch, acacia gum, calcium phosphate, alginate, gelatin, calcium silicate, microcrystalline cellulose, polyvinylpyrrolidone, cellulose, water, syrup, methyl cellulose, methyl hydroxybenzoate, propyl hydroxybenzoate, talc, magnesium stearate, mineral oil, etc., although not being limited thereto. The pharmaceutical composition of the present disclosure may further contain a lubricant, a wetting agent, a sweetener, a flavoring agent, an emulsifier, a suspending agent, a preservative etc. in addition to the above-described ingredients. Suitable pharmaceutically acceptable carriers and formulations are described in detail in Remington's Pharmaceutical Sciences (19th ed., 1995).

The pharmaceutical composition of the present disclosure may be administered orally or parenterally. Specifically, it may be administered parenterally, e.g., by intravenous injection, transdermal administration, subcutaneous injection, intramuscular injection, intravitreal injection, subretinal injection, suprachoroidal injection, eye drop administration, intracerebroventricular injection, intrathecal injection, intraamniotic injection, intraarterial injection, intraarticular injection), intracardiac injection, intracavernous injection, intracerebral injection, intracisternal injection, intracoronary injection, intracranial injection, intradural injection, epidural injection, intrahippocampal injection, intranasal injection, intraosseous injection, intraperitoneal injection, intrapleural injection, intraspinal injection, intrathoracic injection, intrathymic injection, intrauterine injection, intravaginal injection, intraventricular injection, intravesical injection, subconjunctival injection, intratumoral injection, topical injection, etc.

An appropriate administration dosage of the pharmaceutical composition of the present disclosure varies depending on such factors as formulation method, administration type, the age, body weight, sex, pathological condition and diet of a patient, administration time, administration route, excretion rate and response sensitivity, and an ordinarily skilled physician can easily determine and prescribe an administration dosage effective for the desired treatment or prevention.

The composition of the present disclosure may be administered in a pharmaceutically effective amount to a subject who is a target of diagnosis, prevention, alleviation or treatment. The "pharmaceutically effective amount" refers to an amount that exhibits an effect as compared to a negative control group. A total effective amount of the antibody or the fragment thereof of the present disclosure may be administered to a patient as a single dose form or a multiple dose form by a fractionated treatment protocol. A daily administration dosage of the antibody or the fragment thereof of the present disclosure for human is generally 0.0001-100 mg/kg. However, the effective administration dosage of the antibody or the fragment thereof of the present disclosure is determined in consideration of various factors such as the administration route of the pharmaceutical composition, number of administration, the age, body weight, health condition, sex and diet of a patient, the severity of a disease, excretion rate, etc. The effective administration dosage of the antibody or the fragment thereof of the present disclosure may be determined by those having ordinary knowledge in the art in consideration of these factors.

The pharmaceutical composition of the present disclosure may be prepared into a single-dose or multiple-dose formulation by using a pharmaceutically acceptable carrier and/or excipient according to a method that can be easily carried out by those having ordinary knowledge in the art to which the present disclosure belongs. The formulation may be a solution in an oily or aqueous medium, a suspension, an emulsion an extract, a powder, a granule, a tablet or a capsule, and may further contain a dispersant or a stabilizer.

In another aspect, the present disclosure provides a use of the antibody or a fragment thereof, the polynucleotide, the vector or the cell in therapy.

In another aspect, the present disclosure provides a method for treating a disease, which includes a step of administering an effective amount of the antibody or a fragment thereof, the polynucleotide, the vector or the cell to a subject.

In the present disclosure, the term "subject" refers to an individual in need of administration of the composition of the present disclosure, and includes a mammal, a bird, a reptile, an amphibian, a fish, etc. without limitation.

According to a specific exemplary embodiment of the present disclosure, the treating method of the present disclosure further includes a step of administering an antibiotic.

According to a specific exemplary embodiment of the present disclosure, the treating method of the present disclosure includes a step of administering the antibody or a fragment thereof, the polynucleotide, the vector or the cell simultaneously, before or after the administration of the antibiotic.

Specifically, the antibiotic includes gentamycin, ampicillin, kanamycin, chloramphenicol, streptomycin, tetracycline, erythromycin, vancomycin, penicillin, spectinomycin, sulfadiazine and trimethoprim, although not being limited thereto.

According to an exemplary embodiment of the present disclosure, the survival rate of a subject having an infectious disease may be increased by administering the WARS-specific antibody of the present disclosure in combination with an antibiotic or before or after administering an antibiotic.

### [Advantageous Effects]

The features and advantages of the present disclosure may be summarized as follows:
(i) The present disclosure provides an antibody binding specifically to tryptophanyl-tRNA synthetase (WARS) or a fragment thereof.
(ii) The antibody or the fragment thereof of the present disclosure has an excellent ability to bind specifically to WARS with no cross-reactivity with other ARSs and has an excellent ability to detect WARS and inhibit the activity thereof. Therefore, it can be effectively used for diagnosing and/or treating an infectious disease.

### [Brief Description of Drawings]

FIGS. 1a-1b schematically show vectors for converting GKB101 to an IgG antibody. FIG. 1a shows a heavy chain vector, and FIG. 1b shows a light chain vector.
FIGS. 2a-2b show a result of measuring the target-binding ability of GKB101. FIG. 2a shows a result of measuring the binding ability of GKB101 for human and mouse WARS1 using GE Biacore T200, and FIG. 2b shows a result of measuring absorbance by ELISA.
FIGS. 3a-3b show a result of verifying the neutralizing ability of GKB101 *in vitro.* FIG. 3a shows decreased secretion of TNF-α, and FIG. 3b shows decreased secretion of mCXCL2 (homolog of human IL-8).

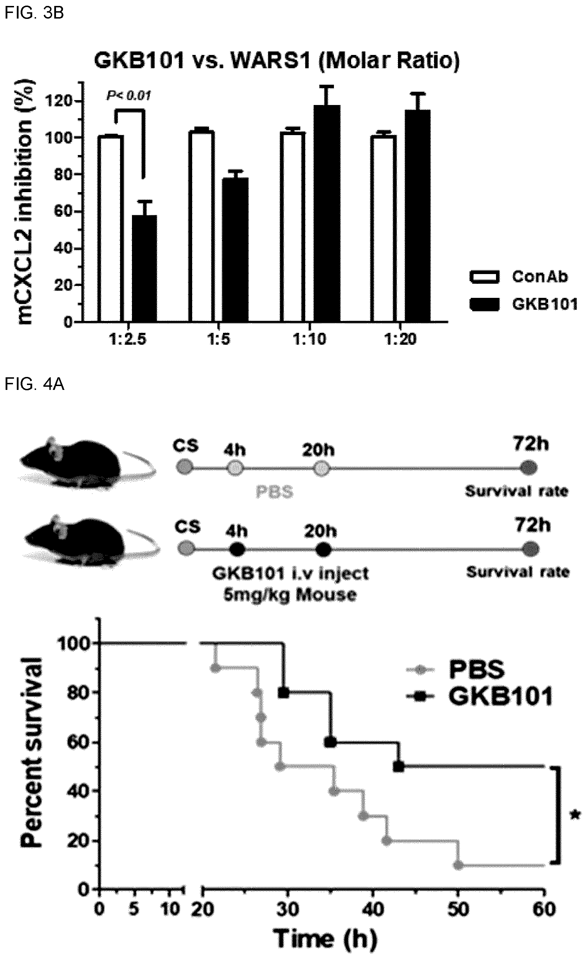

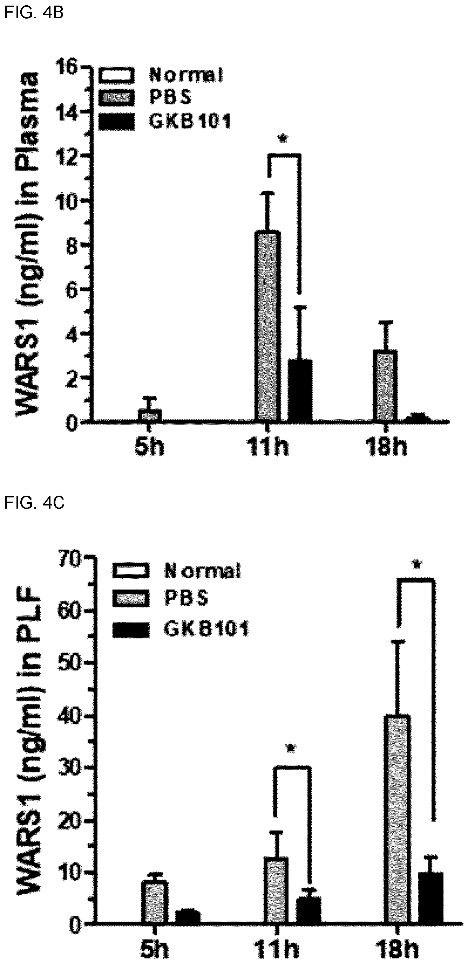

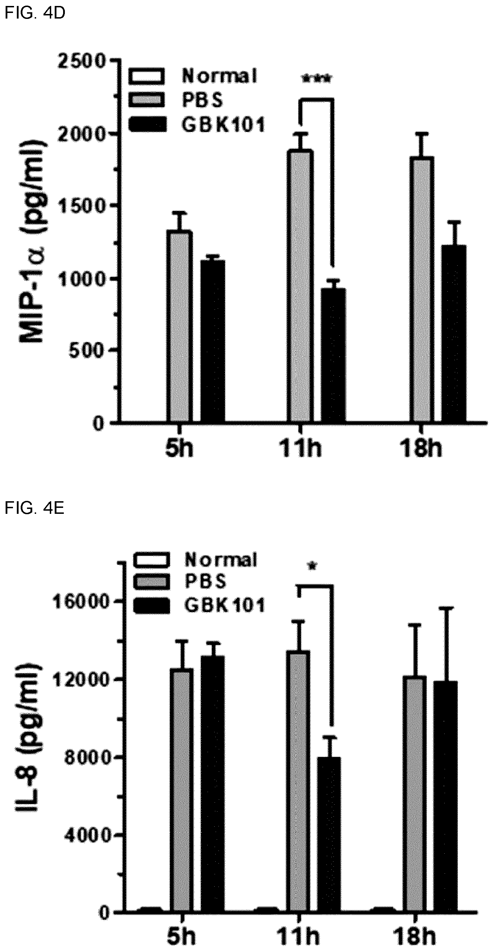

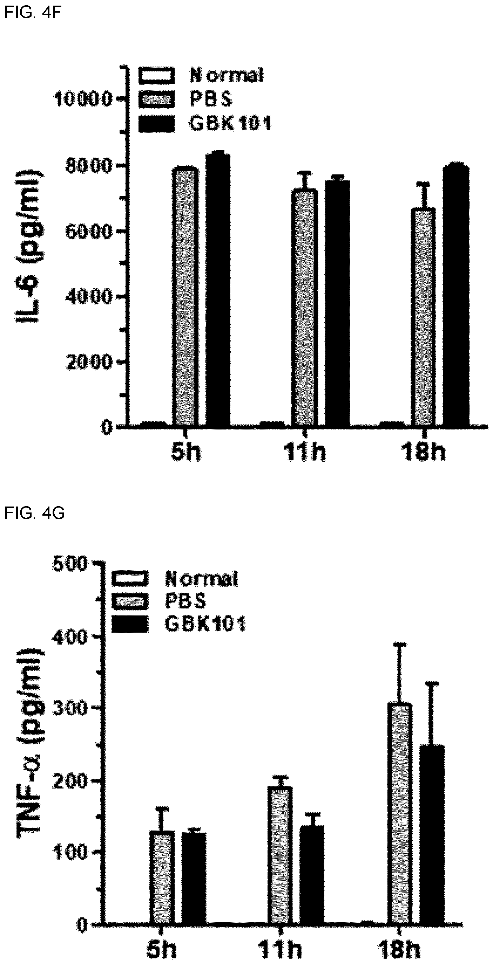

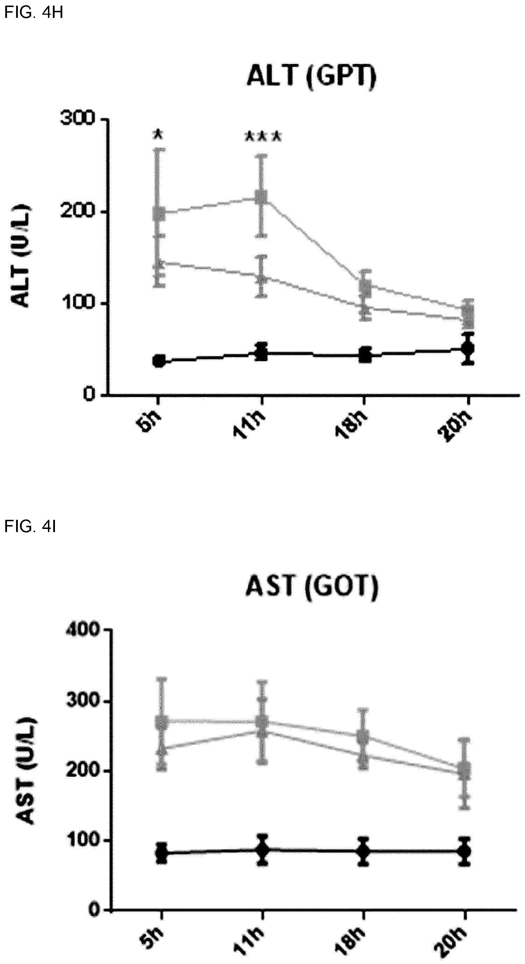

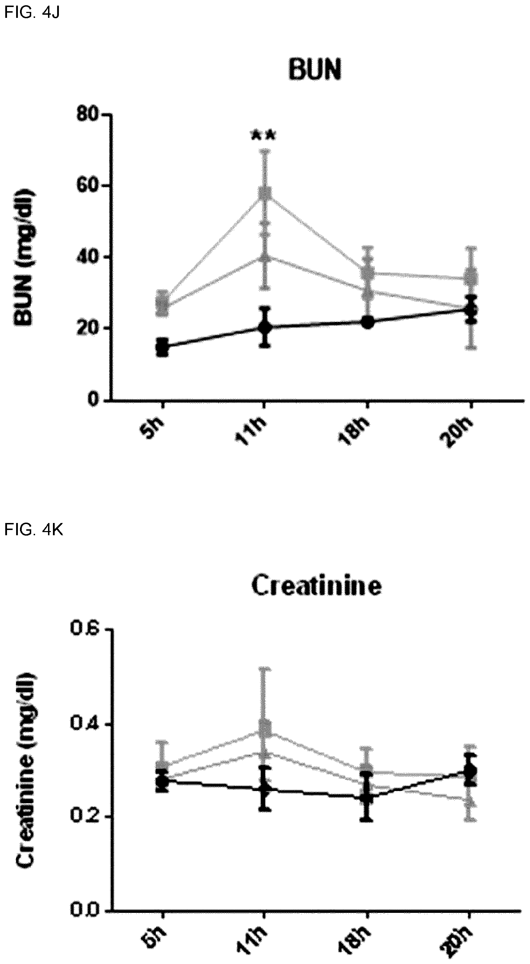

FIGS. 4a-4k show a result of verifying the effect of the WARS1-specific antibody on the survival rate of mice infected by bacteria. FIG. 4a shows a result of injecting PBS or GKB101 to mice in which severe sepsis was induced by intraperitoneal injection of a cecal slurry (CS) and then analyzing survival rate. FIGS. 4b and 4c show a result of verifying that the concentration of WARS1 is decreased by administration of GKB101 (FIG. 4b: plasma, FIG. 4c: peritoneal lavage fluid). FIGS. 4d-4g show a result of verifying that the concentration of IL-8 (FIG. 4e), IL-6 (FIG. 4f), MIP-1α (FIG. 4d) and TNF-α (FIG. 4g) secreted into a peritoneal lavage fluid is decreased by administration of GKB101. FIGS. 4h-4k show a result of verifying that the concentration of ALT (FIG. 4h), AST (FIG. 4i), BUN (FIG. 4j) and creatinine (FIG. 4k) is decreased relatively by administration of GKB101 (circles: normal group; squares: PBS group; triangles GKB101 group).

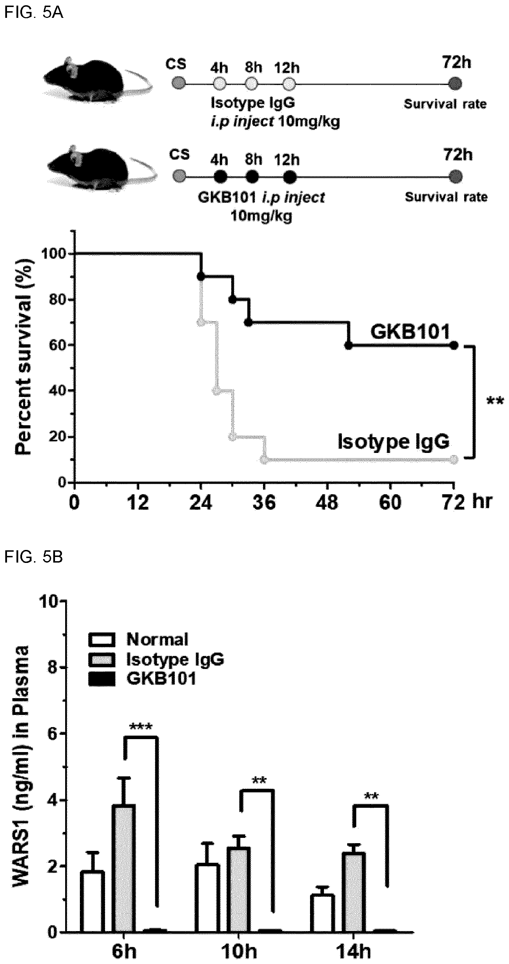

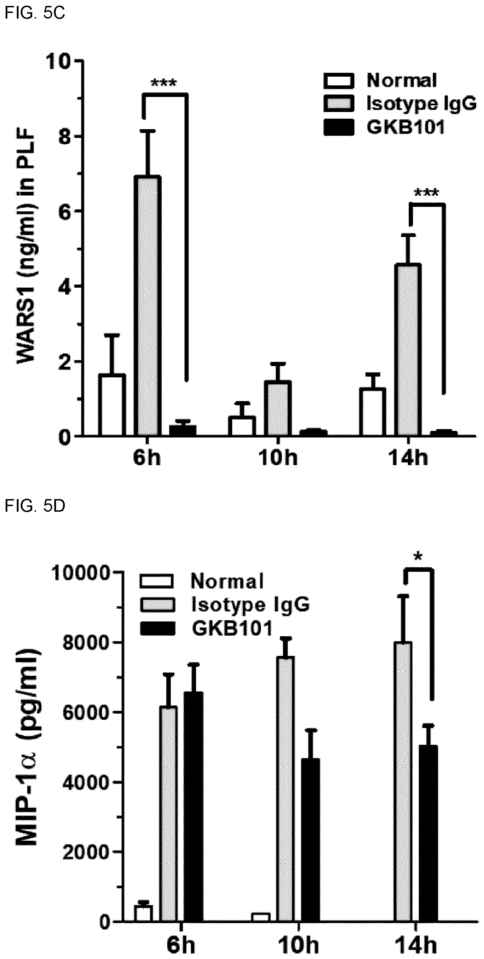

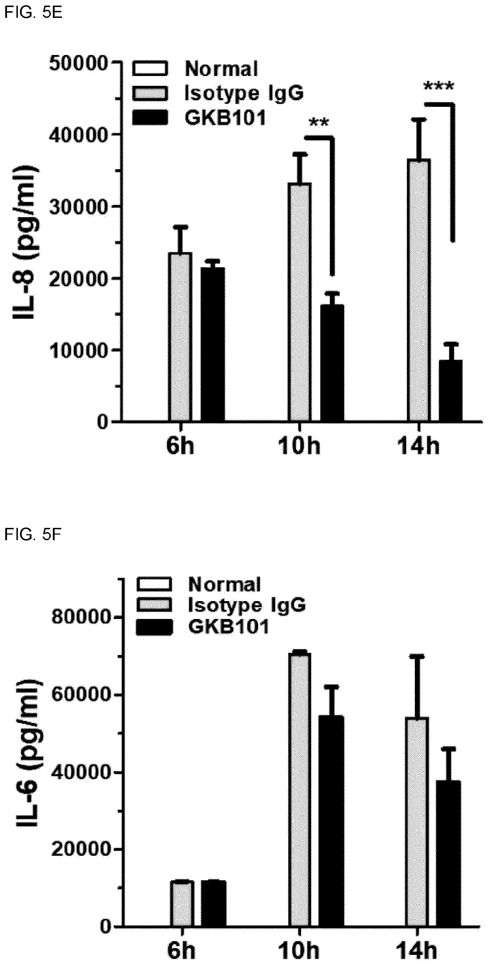

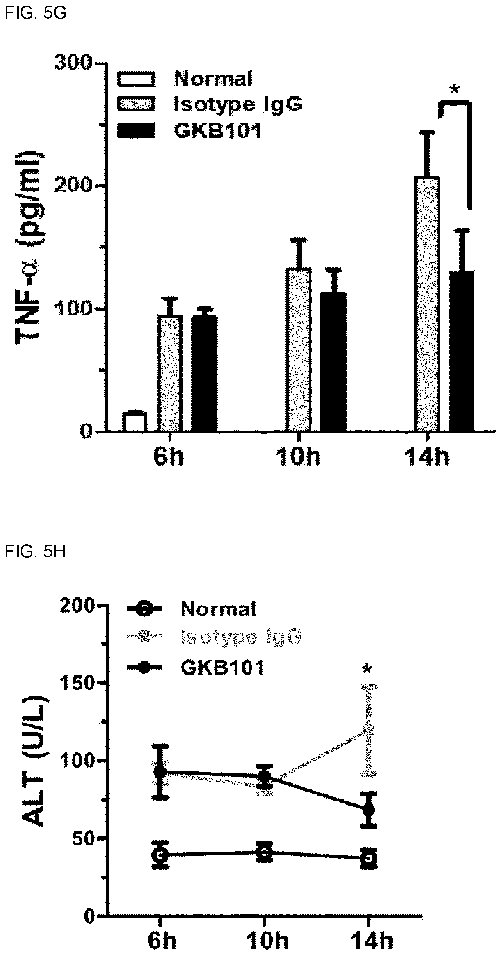

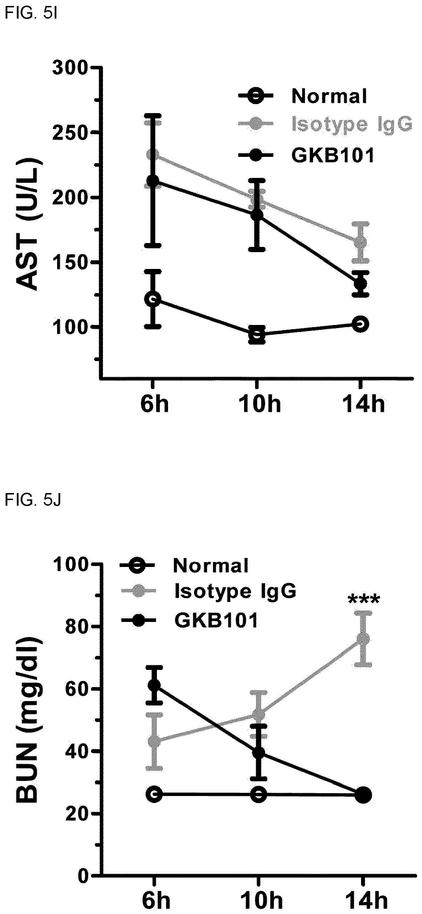

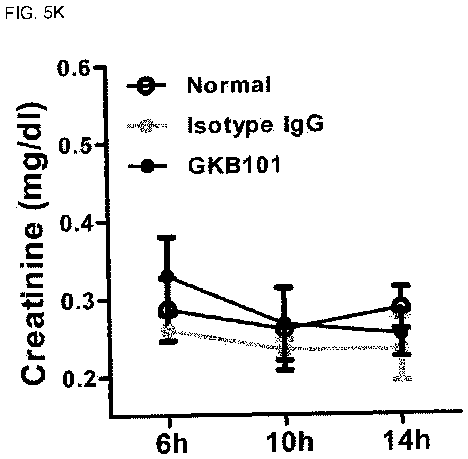

FIGS. 5a-5k show a result of verifying the effect of the WARS1-specific antibody on the survival rate of mice infected by bacteria. FIG. 5a shows a result of injecting isotype IgG or GKB101 to mice in which severe sepsis was induced by intraperitoneal injection of a cecal slurry (CS) and then analyzing survival rate. FIGS. 5b and 5c show a result of verifying that the concentration of WARS1 is decreased by administration of GKB101 (FIG. 5b: plasma, FIG. 5c: peritoneal lavage fluid). FIGS. 5d-5g show a result of verifying that the concentration of IL-8 (FIG. 5e), IL-6 (FIG. 5f), MIP-1α (FIG. 5d) and TNF-α (FIG. 5g) secreted into a peritoneal lavage fluid is decreased by administration of GKB101. FIGS. 5i-5k show a result of verifying that the concentration of ALT (FIG. 5h), AST (FIG. 5i), BUN (FIG. 5j) and creatinine (FIG. 5k) is decreased relatively by administration of GKB101.
FIGS. 6a-6b show a result of verifying the effect of co-administration of GKB101 and an antibiotic in a severe sepsis mouse model. FIG. 6a shows an injection schedule of gentamicin and GKB101, and FIG. 6b shows survival rate over time after injection of gentamicin and GKB101.
FIGS. 7a-7g show a result of verifying the efficacy of GKB101 in a marmoset endotoxemia model. FIG. 7a shows an injection schedule of PBS and GKB101, and FIGS. 7b-7e show the change in body temperature (FIG. 7b), body weight (FIG. 7c), WBC in blood (FIG. 7d) and neutrophil (FIG. 7e) over time in a PBS or GKB101 administration group after LPS induction. FIG. 7f shows the level of WARS1 in plasma after administration of GKB101, and FIG. 7g shows a result of measuring the level of TNF-α.

### [Best Mode]

Hereinafter, the present disclosure will be described more specifically through examples. The following examples are provided only to describe the present disclosure more specifically and it will be obvious to those having ordinary knowledge in the art that the scope of the present disclosure is not limited by the examples.

### Examples

### Example 1. Conversion of scFv antibody to IgG1 antibody

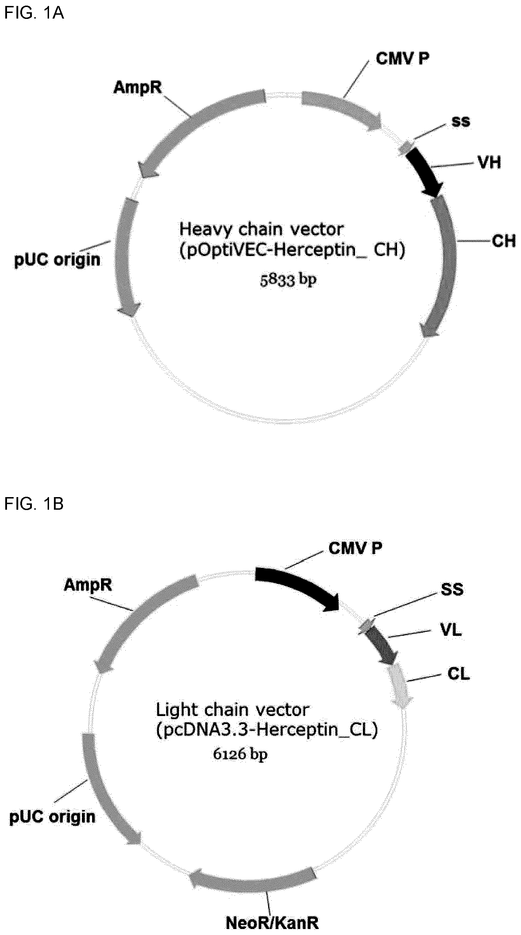

As an scFv antibody binding specifically to WARS1 (SEQ ID NO 26), an scFv antibody including a heavy chain variable region (SEQ ID NO 1) including heavy chain CDR1 (SEQ ID NO 3), heavy chain CDR2 (SEQ ID NO 4) and heavy chain CDR3 (SEQ ID NO 5) and a light chain variable region (V_{L}) of SEQ ID NO 2 including light chain CDR1 (SEQ ID NO 6), light chain CDR2 (SEQ ID NO 7) and light chain CDR3 (SEQ ID NO 8) was selected. The selected scFv antibody was cloned into a plasmid DNA vector by inserting into a heavy chain vector (FIG. 1a) and a light chain vector (FIG. 1b). First, a polynucleotide encoding the scFv was amplified by PCR. The base sequences of the primers used to amplify the CDR regions of the scFv are as follows: forward (gtggccacagcggccgatgtccactcggaagtacagttggtcgaaagtggc, SEQ ID NO 9), reverse (gaagaccgatgggcccttggtgctagccgatgagacggtcactaaagtgcc, SEQ ID NO 10).

The base sequences of the primers used to amplify the V_{L} region of the scFv are as follows: forward (gccacagcggccgatgtccactcggacattcaaatgacgcagagtccctc, SEQ ID NO 11), reverse (gaagacagatggtgcagccacagatcttttaatttccactttagttccctgcc, SEQ ID NO 12).

A monoclonal antibody (hereinafter, referred to as GKB101) was prepared using the scFv antibody by IgG conversion as follows:
A heavy chain and a light chain for IgG conversion of GKB101 were amplified using pOptiVEC and pcDNA 3.3, which are parent vectors expressing herceptin (FIGS. 1a and 1b). The base sequences of the primers used to amplify C_{H} (V_{H}3-23) and C_{L} regions are as follows: C_{H} forward (ggcactttagtgaccgtctcatcggctagcaccaagggcccatcggtcttc, SEQ ID NO 13), C_{H} reverse (gccactttcgaccaactgtacttccgagtggacatcggccgctgtggccac, SEQ ID NO 14), C_{L} forward (ggcagggaactaaagtggaaattaaaagatctgtggctgcaccatctgtcttc, SEQ ID NO 15), C_{L} reverse (gagggactctgcgtcatttgaatgtccgagtggacatcggccgctgtggc, SEQ ID NO 16).

Fab₂ (V_{H}-1-69) for IgG conversion of GKB101 was amplified by PCR using an insert V_{H}1-69_Forward (gtggccacagcggccgatgtccactcgcaagttcagctggtccagagcggc, SEQ ID NO 17), V_{H}1-69_Reverse (gaagaccgatgggcccttggtgctagccgatgagacggtaaccagagtaccc, SEQ ID NO 18) and a vector C_{H}1-69_Forward (gccgctctggaccagctgaacttgcgagtggacatcggccgctgtggccacc, SEQ ID NO 19), C_{H}1-69_Reverse (gggtactctggttaccgtctcatcggctagcaccaagggcccatcggtcttc, SEQ ID NO 20).

The C_{H}, C_{L} and V_{H} 1-69 genes of V_{H}, V_{L} and herceptin expression vectors of GKB101 for GKB101 conversion was amplified by PCR using each vector template (pcDNA3.3- herceptin_C_{L}, pOptiVEC- herceptin_C_{H}) and insert template (scFv, Fab phage vector) and the primers described above (10 pmol each) under the condition of 95 °C/3 min; 95 °C/30 sec, 60 °C/30 sec, 72 °C/30 sec, 30 cycles; 72 °C/5 min. The PCR product was inserted into a vector used for IgG production using a Dpnl restriction enzyme. The prepared vector including a DNA which encodes the light chain and heavy chain of IgG including the variable region of the scFv was co-transfected into Expi293F cells so that the light chain and the heavy chain were co-expressed in the cells.

### Example 2. Transient transfection and purification using column

ExpiHEK293 cells (Thermofisher) and a Freestype293 expression medium (Thermofisher) were used for purification of proteins using a transient expression system. Transfection was conducted using an Expifectamine 293 reagent (Thermofisher) and a plasmid DNA with the heavy chain and the light chain of GKB101 at 1:1. After the transfection, the cells were cultured for about 5 days under shaking. After centrifuging the cell culture, only the supernatant was recovered and used for isolation and purification of proteins. The recovered supernatant was loaded in a Hitrap MabselectSure column (GE Healthcare Life Sciences). After removing nonspecifically bound proteins by washing with PBS, proteins specifically bound to the column were separated using a 100 mM citrate buffer (pH 3.0) + 50 mM NaCl. High-purity GKB101 was obtained by loading an eluate obtained by FPLC in a size-exclusion chromatography system (GE Healthcare Life Sciences). The final yield of GKB101 obtained by optimizing the condition of the transient expression system was 37 mg for 500 mL of the cell culture and the yield of the endotoxin was about 0.002 EU/g.

The sequences of the prepared GKB101 antibody are as follows:

**[Table 1]**

| | Sequences of GKB101 antibody | |
|---|---|---|
| | | Amino acid sequence |
| V_{H} (SEQ ID NO 1) | | EVQLLESGGG LVQPGGSLRL SCAASGFTFS SYDMSWVRQA PGKGLEWVSA ISSGGSSIYY ADSVKGRFTI SRDNSKNTLY LQMNSLRAED TAVYYCARDV AWDMLGDFDY WGQGTLVTVS S |
| V_{L} (SEQ ID NO 2) | | QSVLTQPPSA SGTPGQRVTI SCTGSSSNIG SNYVYWYQQL PGTAPKLLIY ANSHRPSGVP DRFSGSKSGT SASLAISGLR SEDEADYYCG AWDDSLSAYV FGGGTKLTV |
| C_{L} (SEQ ID NO 21) | | TVAAPSVFIFPPSDEQLKSGTASVVCLLNNFYPREAKVQWK |
| | | VDNALQSGNSQESVTEQDSKDSTYSLSSTLTLSKADYEKH KVYACEVTHQGLSSPVTKSFNRGEC |
| | C_{H}1 (SEQ ID NO 22) | ASTKGPSVFPLAPSSKSTSGGTAALGCLVKDYFPEPVTVS WNSGALTSGVHTFPAVLQSSGLYSLSSVVTVPSSSLGTQT YICNVNHKPSNTKVDKKV |
| | Hinge (SEQ ID NO 23) | EPKSCDKTHTCPPCPAPELLGGP |
| | C_{H}2 (SEQ ID NO 24) | SVFLFPPKPKDTLMISRTPEVTCVVVDVSHEDPEVKFNWYV DGVEVHNAKTKPREEQYNSTYRVVSVLTVLHQDWLNGKE YKCKVSNKALPAPIEKTISKAKG |
| | C_{H}3 (SEQ ID NO 25) | REPQVYTLPPSREEMTKNQVSLTCLVKGFYPSDIAVEWES NGQPENNYKTTPPVLDSDGSFFLYSKLTVDKSRWQQGNV FSCSVMHEALHNHYTQKSLSL |

### Example 3. Measurement of target-binding ability of GKB101

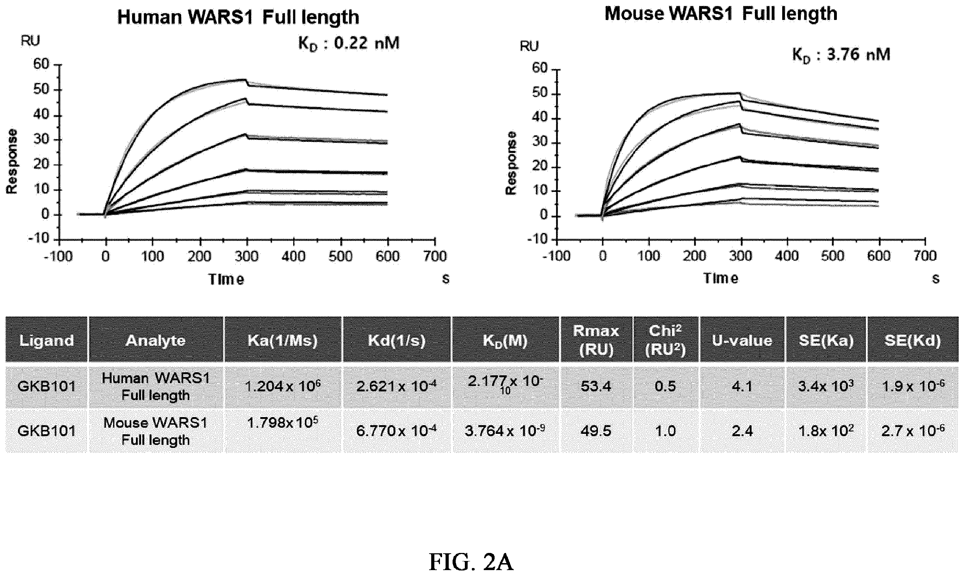

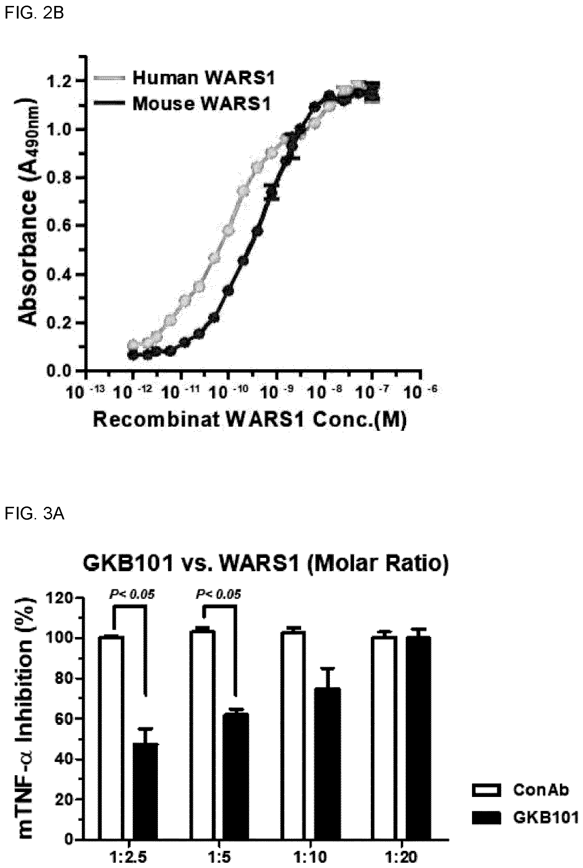

1) SPR: Binding ability was measured using Series S Sensor CM5 Chip (GE Healthcare Life Sciences) and GE Bioacore T200. A HBS-EP buffer (GE Healthcare Life Sciences) was used. After injecting serially diluted GKB101 or buffer for 1-10 minutes at a flow rate of 30 µL/min, the analyte was washed for 20 minutes.
2) ELISA: After coating the GKB101 on a 96-well plate (Maxisorp, Thermofisher) at a concentration of 1 µg/mL, it was blocked at room temperature for 1 hour with PBS containing 1% BSA. After diluting the recombinant WARS1 protein to different concentrations and adding to each coated well, reaction was conducted at room temperature for 1 hour and each well was washed 4 times with PBST (PBS + Tween 20). After adding anti-WARS1 antibody (Abfrontier) to each well and conducting reaction at room temperature for 1 hour, each well was washed 4 times with PBST. After reacting with HRP-conjugated secondary antibody (anti-Human IgG, Cell Signaling) for 1 hour, followed by washing with PBST, absorbance was measured at 490 nm (VersaMax microplate reader) after reacting with a TMB substrate (BD).
3) Immunoblot: After loading 20 ng of each of WARS1 human and mouse recombinant proteins and 30 µg of each of THP-1 human monocytes and J774.1A mouse macrophages, stimulated with LPS for 24 hours, on 8% SDS-PAGE, the cells were transferred to a PVDF membrane (Millipore). The membrane was reacted with 1 µg/mL GKB101 for 1 hour and then washed three times with TBST (TBS + Tween 20). After reacting with a secondary antibody (anti-human IgG HRP, Millipore) for an hour, followed by washing 3 times with TBST, the membrane was reacted with ECL (West^{®} bright ECL, Advanta) (LAS-4000, Fujifilm).
4) Result: As a result of measuring the binding ability of GKB101 for the recombinant WARS1 proteins, it showed strong binding ability for human WARS1 in both ELISA and SPR. The binding ability was about 0.2 nM for the human recombinant protein and about 3.76 nM for the mouse recombinant protein (FIG. 2a). In addition, WARS1 in the culture of human monocytes and mouse macrophages could also be detected effectively with GKB101 in the immunoblot experiment (FIG. 2b).

### Example 4. Verification of neutralizing ability of WARS1-specific antibody in vitro

The neutralizing ability of the WARS1-specific antibody GKB101 *in vitro* was investigated using J774.1A mouse macrophages. The mouse macrophages were cultured using high-glucose DMEM (GIBCO) supplemented with 10% FBS and 1% penicillin/streptomycin. 8×10⁴ cells were seeded on a 48-well cell culture plate (Nunc) and the medium was replaced with plain DMEM 2 hours before treating the cells with an antigen-antibody mixture. After mixing 200 nM recombinant protein WARS1 with GKB101 of different concentrations and reacting in a 37 °C incubator for 2 hours, the mixture was added to the cells seeded on each well. About 15 hours later, the cell culture was centrifuged and the supernatant was used to measure CXCL2 (R&D) and TNF-α (Biolegend). ELISA was conducted according to the manufacturers' instructions.

In the mouse macrophages, GKB101 resulted in significantly decreased secretion of TNF-α in a concentration-dependent manner with respect to 200 nM WARS1 at 1:2.5 and 1:5 (FIG. 3a). mCXCL2 (mouse IL-8 homolog) was decreased at 1:5 and 1:2.5, and statistically significant difference was observed at 1:2.5 (FIG. 3b). All the results were analyzed statistically by ANOVA.

### Example 5. Effect of WARS1-specific antibody on survival rate of mice infected by bacteria

After inducing severe sepsis by injecting CS into mice, 2.5 mg/kg PBS or GKB101 was injected i.v twice at 4 hours or 20 hours after the intraperitoneal injection of the CS. Then, survival rate was monitored until 60 hours.

WARS1 and cytokine assay: In order to investigate the neutralizing ability of GKB101 for WARS1, peritoneal lavage fluid and blood were taken at 5 hours, 11 hour, 18 hours and 20 hours, and the level of WARS1 (in-house), TNF-α (Biolegend), MIP-1α (R&D), mIL-6 (Biolegend) and mCXCL2 (IL-8 homolog, R&D) was measured by ELISA. The ELSIA for the cytokines was conducted according to the manufacturers' instructions except for WARS1. WARS1 level was measured as follows. After coating GKB101 onto each well of a 96-well plate (Maxisorp, Thermofisher) at 1 µg/mL, the well was blocked at room temperature for 1 hour with PBS containing 1% BSA. After adding diluted standard plasma, peritoneal lavage fluid sample and recombinant mouse recombinant protein to the blocked well, reaction was conducted at room temperature for 1 hour and the well was washed 4 times with PBST (PBS + Tween 20). After adding anti-WARS1 antibody (Abfrontier) and conducting reaction at room temperature for 1 hour, each well was washed 4 times with PBST. After reacting with HRP-conjugated secondary antibody (anti-human IgG, Cell Signaling) for 1 hour, the well was washed with PBST. After reacting with a TMB substrate (BD), absorbance was measured at 490 nm (VersaMax microplate reader).

Biochemical assay using blood: After injecting some of blood into a vacutainer holding a clot activator, the blood was coagulated by leaving alone at room temperature for 10-15 minutes. After centrifuging at 3,000 rpm for 10 minutes, the level of aspartate aminotransferase (AST), alanine aminotransferase (ALT), blood urea nitrogen (BUN) and creatinine in the obtained serum was measured using a biochemical blood analyzer (7180 Hitachi, Japan).

In order to investigate the effect of GKB101 on the survival rate of mice, animal experiment was conducted as described schematically in FIG. 4a. PBS or GKB101 was injected i.v at 4 hours and 20 hours after induction of severe sepsis by intraperitoneal injection of a cecal slurry (CS). The survival rate of the mice was monitored until 60 hours after the infection. As a result, for CS + PBS (PBS was injected after induction of severe sepsis), the survival rate was 50% at 29 hours and 20% at 41 hours. For CS+GKB101 (GKB101 was injected after induction of severe sepsis), the survival rate was increased as compared to the PBS group, as 80% at 29 hours and 50% at 41 hours. The concentration of WARS1 in the peritoneal lavage fluid (FIG. 4c) and plasma (FIG. 4b) was decreased significantly by GKB101 at 11 hours and 18 hours, and the concentration of IL-8 (FIG. 4e), IL-6 (FIG. 4f), MIP-1α (FIG. 4d) and TNF-α (FIG. 4g) secreted into the peritoneal lavage fluid was also decreased significantly at 11 hours and 18 hours. The concentration of ALT (FIG. 4h), AST (FIG. 4i), BUN (FIG. 4j) and creatinine (FIG. 4k) was decreased relatively, and the concentration of ALT and BUN was decreased significantly at 11 hours in the GKB101 injection group as compared to the PBS group. All the results were analyzed statistically by ANOVA (*, p < 0.05; **, p < 0.01; ***, p < 0.001).

### Example 6. Effect of WARS1-specific antibody on survival rate of mice infected by bacteria 2

After inducing severe sepsis by injecting CS to mice, 10 mg/kg isotype IgG or GKB101 was injected i.p three times at 4 hours, 8 hours and 12 hours as 5 mg/kg, 2.5 mg/kg and 2.5 mg/kg, respectively. Then, survival rate was monitored until 72 hours.

WARS1 and cytokine assay: In order to investigate the neutralizing ability of GKB101 for WARS1, peritoneal lavage fluid and blood were taken at 6 hours, 10 hours and 14 hours, and the level of WARS1 (in-house), TNF-α (Biolegend), MIP-1α (R&D), mIL-6 (Biolegend) and mCXCL2 (IL-8 homolog, R&D) was measured by ELISA. The ELSIA for the cytokines was conducted according to the manufacturers' instructions except for WARS1. WARS1 level was measured as follows. After coating GKB101 onto each well of a 96-well plate (Maxisorp, Thermofisher) at 1 µg/mL, the well was blocked at room temperature for 1 hour with PBS containing 1% BSA. After adding diluted standard plasma, peritoneal lavage fluid sample and recombinant mouse recombinant protein to the blocked well, reaction was conducted at room temperature for 1 hour and the well was washed 4 times with PBST (PBS + Tween 20). After adding anti-WARS1 antibody (Abfrontier) and conducting reaction at room temperature for 1 hour, each well was washed 4 times with PBST. After reacting with HRP-conjugated secondary antibody (anti-human IgG, Cell Signaling) for 1 hour, the well was washed with PBST. After reacting with a TMB substrate (BD), absorbance was measured at 490 nm (VersaMax microplate reader).

In order to investigate the effect of GKB101 on the survival rate of mice, animal experiment was conducted as described schematically in FIG. 5a. Isotype IgG or GKB101 was injected i.p at 4 hours, 8 hours and 12 hours after induction of severe sepsis by intraperitoneal injection of a cecal slurry (CS). The survival rate of the mice was monitored until 72 hours after the infection. As a result, for CS + isotype IgG (isotype IgG was injected after induction of severe sepsis), the survival rate was 20% at 28 hours and 10% at 36 hours. For CS + GKB101 (GKB101 was injected after induction of severe sepsis), the survival rate was increased remarkably as compared to the isotype IgG group, as 80% at 28 hours and 70% at 36 hours (FIG. 5a). The concentration of WARS1 in the peritoneal lavage fluid (FIG. 5c) and plasma (FIG. 5b) was decreased significantly by GKB101 at 6 hours, 10 hours and 14 hours, and the concentration of IL-8 (FIG. 5e), IL-6 (FIG. 5f), MIP-1α (FIG. 5d) and TNF-α (FIG. 5g) secreted into the peritoneal lavage fluid was also decreased after the injection of GKB101. In particular, the concentration of IL-8, MIP-1α and TNF-α was decreased significantly at 14 hours. The concentration of ALT (FIG. 5h), AST (FIG. 5i), BUN (FIG. 5j) and creatinine (FIG. 5k) was decreased relatively, and the concentration of ALT and BUN was decreased significantly at 14 hours in the GKB101 injection group as compared to the isotype IgG group. All the results were analyzed statistically by ANOVA (*, p < 0.05; **, p < 0.01; ***, p < 0.001).

### Example 7. Effect of WARS1-specific antibody and antibiotic on survival rate of mice infected by bacteria

After administering an antibiotic (gentamicin) to mice in which severe sepsis had been induced by injecting CS, 10 mg/kg isotype IgG or GKB101 was injected i.v at 4 hours, 8 hours and 20 hours as 5 mg/kg, 2.5 mg/kg and 2.5 mg/kg, respectively. Then, survival rate was monitored until 72 hours.

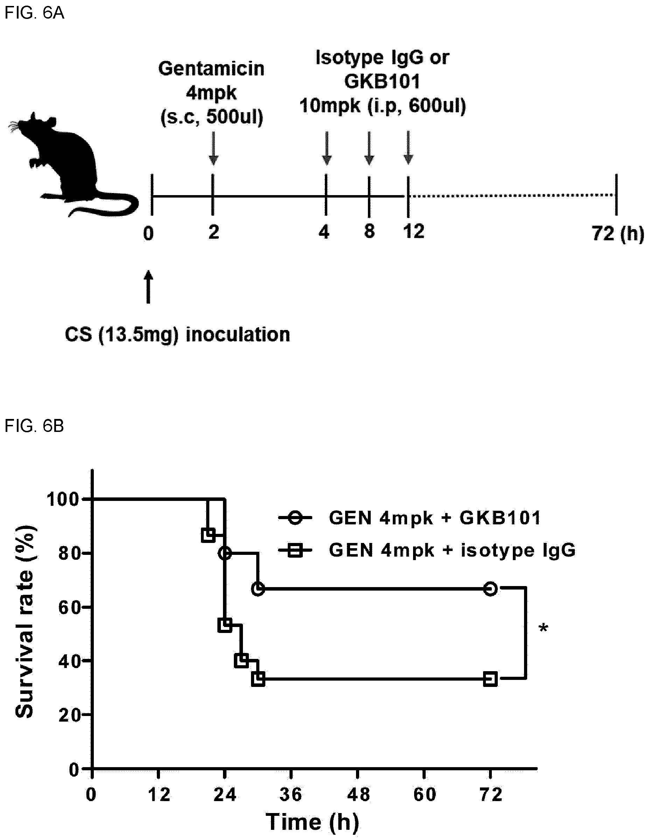

As a result of analyzing the survival rate in the CS-induced mice to which GKB101 and the antibiotic were co-administered, significantly difference in survival rate was observed with 80% for the GKB101 administration group at 24 hours and 70% for the isotype IgG administration group at 72 hours. All the results were analyzed statistically by ANOVA (FIG. 6).

### Example 8. Verification of efficacy of GKB101 using marmoset endotoxemia model

Marmosets (*Callithrix jacchus)* were acquired from the Laboratory Animal Center of Osong Medical Innovation Foundation (Chungbuk, Korea). All the common marmosets were housed in stainless steel cages. They were maintained in 12-hour dawn and 12-hour dusk conditions at a temperature of 27±2 °C and a humidity of 30%-70% in the SPF animal facility. The common marmosets were provided with a standard marmoset diet (#0639; Altromin, Lage, Germany) and water *ad libitum.* All protocols were approved by the Committee on Animal Care of the Laboratory Animal Center of Osong Medical Innovation Foundation. After injecting LPS to the marmosets and inducing endotoxemia, 2 mg/kg PBS or GKB101 was injected i.v twice at 0.2 hours and 2 hours and the animals were monitored for 24 hours.

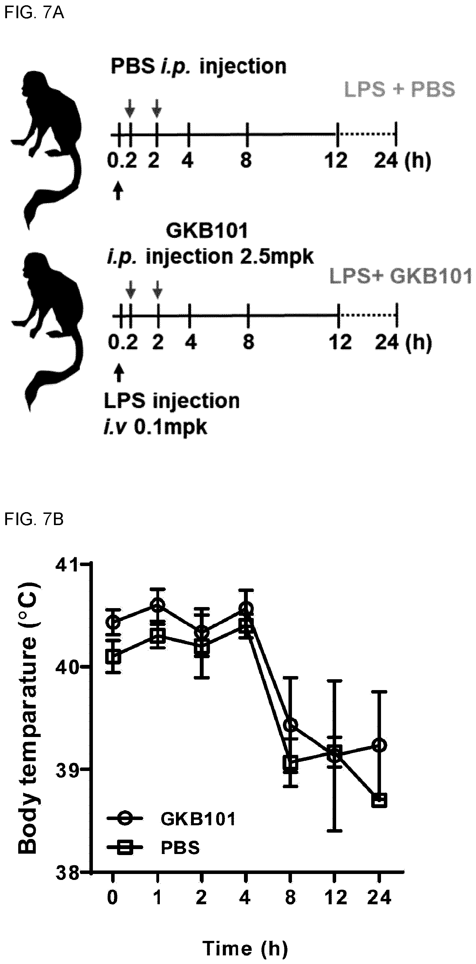

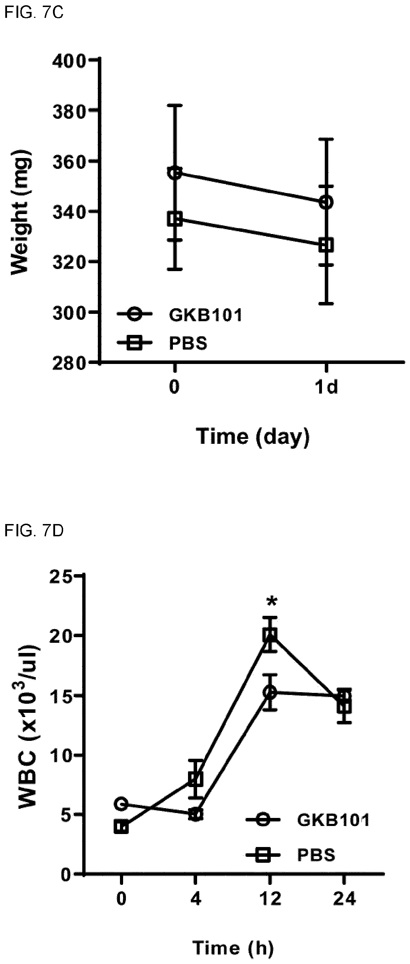

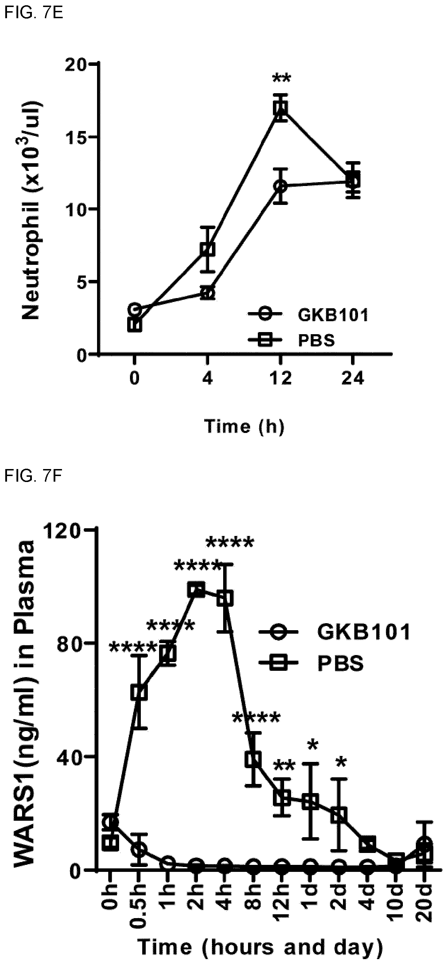

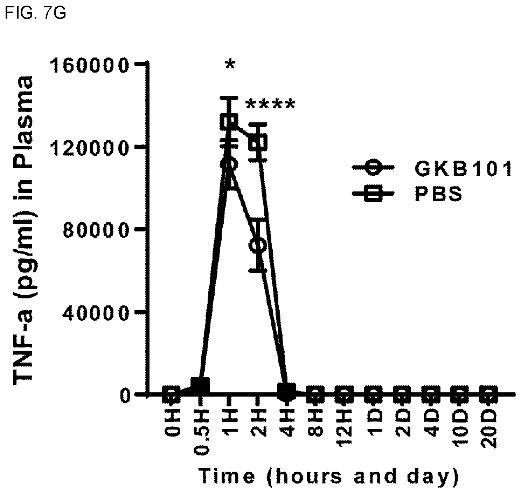

As a result of investigating the number of immune cells in the blood of the marmoset endotoxemia model, the group to which the anti-WARS1 antibody was administered showed significant increase of WBCs and neutrophils at 12 hours (*, p < 0.05, **, p < 0.01). In addition, as a result of comparing the effect of the administration of GKB101 on the expression of WARS1, cytokines and chemokines in blood, WARS1 was decreased significantly at 0.5, 1, 2, 4 and 8 hours (****, p < 0.0001) and TNF-α was decreased significantly at 1 and 2 hours (2 hours, ****, p < 0.0001) in the GKB101 administration group. All the results were analyzed statistically by ANOVA (FIGS. 7a-7g).

The results of the above-described examples verify that GKB101 has a strong binding ability to WARS1 and has the neutralizing ability of decreasing the expression of cytokines expressed by WARS1 *in vivo* and *in vitro.* As described above, it is expected that the composition of the present disclosure is industrially applicable as an antibody drug which specifically detects WARS1, which induces hyperinflammation in the early stage of infection, and inhibits the same.

Although the examples of the present disclosure have been described above, those having ordinary knowledge in the art can change and modify the present disclosure variously through addition, change or deletion without departing from the scope of the present disclosure defined by the appended claims.

## Claims

1. An antibody binding specifically to tryptophanyl-tRNA synthetase (WARS), comprising a heavy chain variable domain (V_{H}) of SEQ ID NO 1 and a light chain variable domain (V_{L}) of SEQ ID NO 2 (V_{L}), or a fragment thereof.

2. An antibody binding specifically to WARS, comprising a heavy chain variable domain (V_{H}) comprising a heavy chain CDR1 of SEQ ID NO 3, a heavy chain CDR2 of SEQ ID NO 4 and a heavy chain CDR3 of SEQ ID NO 5 and a light chain variable domain (V_{L}) comprising a light chain CDR1 of SEQ ID NO 6, a light chain CDR2 of SEQ ID NO 7 and a light chain CDR3 of SEQ ID NO 8, or a fragment thereof.

3. The antibody or the fragment thereof according to claim 1 or 2, wherein the antibody or the fragment thereof comprises one or more sequence selected from a group consisting of a C_{L} sequence of SEQ ID NO 21, a C_{H}1 sequence of SEQ ID NO 22, a hinge sequence of SEQ ID NO 23, a C_{H}2 sequence of SEQ ID NO 24 and a C_{H}3 sequence of SEQ ID NO 25.

4. The antibody or the fragment thereof according to claim 1 or 2, wherein the fragment is a fragment selected from a group consisting of Fab, F(ab')₂, Fd, sdFv, Fv, dAb, scFv, sdAb and a tetramer or the fragment bound to Fc.

5. A polynucleotide encoding the antibody or the fragment thereof according to claim 1 or 2.

6. A vector comprising the polynucleotide according to claim 5.

7. A cell transformed with the vector according to claim 6.

8. A method for preparing an antibody binding specifically to WARS or a fragment thereof, comprising a step of culturing the cell according to claim 7 under a condition where a polynucleotide is expressed and recovering the polypeptide.

9. A method for detecting WARS specifically, comprising a step of contacting the antibody or the fragment thereof according to claim 1 or 2, a polynucleotide encoding the antibody or the fragment thereof, a vector comprising the polynucleotide or a cell transformed with the vector with a sample.

10. A composition for detecting WARS specifically, comprising the antibody or the fragment thereof according to claim 1 or 2, a polynucleotide encoding the antibody or the fragment thereof, a vector comprising the polynucleotide or a cell transformed with the vector.

11. A composition for diagnosing an infectious disease, comprising the antibody or the fragment thereof according to claim 1 or 2, a polynucleotide encoding the antibody or the fragment thereof, a vector comprising the polynucleotide or a cell transformed with the vector.

12. The composition for diagnosing according to claim 11, wherein the infectious disease is an infectious inflammatory disease.

13. The composition for diagnosing according to claim 12, wherein the infectious inflammatory disease is sepsis or septic shock.

14. A pharmaceutical composition for preventing or treating an infectious disease, comprising the antibody or the fragment thereof according to claim 1 or 2, a polynucleotide encoding the antibody or the fragment thereof, a vector comprising the polynucleotide or a cell transformed with the vector.

15. The pharmaceutical composition for preventing or treating an infectious disease according to claim 14, wherein the pharmaceutical composition further comprises an antibiotic.

16. The pharmaceutical composition for preventing or treating an infectious disease according to claim 14, wherein the pharmaceutical composition is administered simultaneously, before or after the administration of the antibiotic.

17. The pharmaceutical composition for preventing or treating an infectious disease according to claim 14, wherein the infectious disease is an infectious inflammatory disease.

18. The pharmaceutical composition for preventing or treating an infectious disease according to claim 17, wherein the infectious inflammatory disease is sepsis or septic shock.

19. A method for treating a disease, comprising a step of administering an effective amount of the antibody or the fragment thereof according to claim 1 or 2, a polynucleotide encoding the antibody or the fragment thereof, a vector comprising the polynucleotide or a cell transformed with the vector to a subject.

20. The method for treating a disease according to claim 19, which comprises a step of further administering an antibiotic.

21. The method for treating a disease according to claim 20, wherein the antibiotic is selected from a group consisting of gentamycin, ampicillin, kanamycin, chloramphenicol, streptomycin, tetracycline, erythromycin, vancomycin, penicillin, spectinomycin, sulfadiazine and trimethoprim.

22. The method for treating a disease according to claim 19, wherein the disease is an infectious disease.

23. The method for treating a disease according to claim 22, wherein the infectious disease is an infectious inflammatory disease.

24. The method for treating a disease according to claim 23, wherein the infectious inflammatory disease is sepsis or septic shock.

25. A use of the antibody or the fragment thereof according to claim 1 or 2, a polynucleotide encoding the antibody or the fragment thereof, a vector comprising the polynucleotide or a cell transformed with the vector in therapy.

26. The use in therapy according to claim 25, wherein the use in therapy is for treating an infectious disease.

27. The use in therapy according to claim 26, wherein the infectious disease is an infectious inflammatory disease.

28. The use in therapy according to claim 27, wherein the infectious inflammatory disease is sepsis or septic shock.
